# EUROPEAN PATENT SPECIFICATION

(11) **EP 4 047 539 B1**
(45) Date of publication and mention of the grant of the patent: **02.04.2025**
(21) Application number: 22157016.1
(22) Date of filing: 16.02.2022
(51) Int. Cl.: G06Q 30/02

(54) **METHOD FOR SAMPLE TEST COUNTING**
VERFAHREN ZUM ZÄHLEN VON PROBENTESTS
PROCÉDÉ DE COMPTAGE DE TESTS D'ÉCHANTILLON

(30) Priority: 22.02.2021 US 202117181820
(43) Date of publication of application: 24.08.2022
(73) Proprietor: SYSMEX CORPORATION, Kobe-shi, Hyogo 651-0073 (JP)
(72) Inventor: Postma, Stephen J., Kobe-shi, Hyogo 651-0073 (JP)
(74) Representative: Hoffmann Eitle

(56) References cited:
- EP-A2- 2 381 379

## Description

### TECHNICAL FIELD

This disclosure relates to systems and methods for determining a count of sample test results that are reportable.

### BACKGROUND OF THE INVENTION

In certain arrangements, a supplier may provide a sample analyzer (possibly also including various supplies and support for the sample analyzer) to a user's facility. The supplier may then charge the user according to the number of tests performed by the sample analyzer. In billing systems for those sample analyzers, not all sample measurements performed by the sample analyzer may be billed. Instead, only sample measurements that are agreed upon between the device provider and the user (e.g., by contractual obligations) may be billed. In such implementations, even if the agreement or contract between the analyzer provider and the user is complicated, it is necessary to accurately identify the sample measurements that are to be charged and reflect those sample measurements in the charged amount.

Existing billing systems may charge a user according to the number of times the analysis server provides analysis data to the user. In such systems, when a sample analyzer installed in a user's facility measures a sample, the measurement data is transmitted to an analysis server via a network, and the analysis server analyzes the measurement data and uses the analysis data. The billing amount is then determined according to the number of times the analysis data is provided.

However, the existing billing systems may charge uniformly for sample measurements that are the source of the analysis data, and any sample measurement performed by the sample analyzer may be charged. Existing billing systems do not judge whether such sample measurements actually exist, or whether such sample measurements should be billed. Therefore, existing billing systems cannot accurately specify the sample measurement to be billed in consideration of various factors.

Accordingly, there is a demand for a new billing system that can accurately identify the sample measurements to be billed and accurately reflect those billable sample measurements in the charged amount. Further, there is a need to provide such sample measurement to a medical doctor. EP 2 381 379 A2 discloses a sample analyzer for analysing patient samples based on user selected tests.

### SUMMARY OF THE INVENTION

In accordance with various embodiments, a method and system for billing for sample tests is disclosed. The billing method takes into account various factors and data in order to provide an accurate billing amount that properly reflects the billable test results.

In one embodiment, a method includes receiving, via network, a plurality of test results from at least one sample analyzer of each of a plurality of customers, selecting test results from the plurality of test results based on a plurality of determination rules adaptable to each of the customers, and determining, for each of the customers, a number of the selected test results for billings to each of the customers. The determination rules may be adaptably defined for each of the customers. The determination rules may be adaptably defined to determine, for each of the customers, whether to select or exclude the test result. The determination rules may be adaptably defined to determine, for each of the customers, which type of test is subjected to a target of the billing.

In certain embodiments, the determination rules are for selecting the test result which is reportable to at least one of a medical doctor or a billing entity, or are for excluding a test result which is not reportable to a medical doctor or a billing entity. In such an instance, the method may further include determining, according to the determination rules for excluding a test result, that a first test result of the plurality of test results is not to be excluded, and incrementing a count number corresponding to a number of reportable test results.

Specifically, the determination rules may include a first rule for selecting the test result of a sample collected from a subject, wherein the first rule is for excluding the test result of a quality control sample, a calibrator sample or a fluid for background check. The determination rules may also include a second rule for excluding the test result which is associated with an error. The determination rules may also include a third rule for excluding the test result which is a duplicate of a retesting, or for excluding the test result whose test item is the same as that of the retesting. The determination rules may also include a fourth rule for selecting the test results based on one or more test items subjected to a target of the billing. With such a fourth rule, determining the number of the selected test results may include determining numbers of the selected test results for each of the test items or a group of the test items.

In another embodiment, a computing system comprises a controller connected to at least one sample analyzer of each of a plurality of customers via network. The controller may be programmed to receive a plurality of test results from the at least one sample analyzer, select test results from the plurality of test results, based on a plurality of determination rules adaptable to each of the customers, and determine, for each of the customers, a number of the selected test results for billings to each of the customers.

In another embodiment, a system includes at least one sample analyzer of each of a plurality of customers, and a computing system connected to the at least one sample analyzer via network. The computing system may be programmed to receive a plurality of test results from the at least one sample analyzer, select test results from the plurality of test results, based on a plurality of determination rules adaptable to each of the customers, and determine, for each of the customers, a number of the selected test results for billings to each of the customers.

### BRIEF DESCRIPTION OF THE DRAWINGS

FIG. 1 is a block diagram of an exemplary system configuration in accordance with various embodiments;
FIG. 2 is a block diagram of an exemplary computing system in accordance with various embodiments;
FIG. 3 is a block diagram of an exemplary data structure in a database in accordance with various embodiments;
FIG. 4 is a block diagram of an exemplary data structure of contract between a customer and a provider in accordance with various embodiments;
FIG. 5 is a block diagram of an exemplary data structure of a measurement result in accordance with various embodiments;
FIG. 6 is a flow diagram of an exemplary algorithm for test report calculation in accordance with various embodiments;
FIG. 7 is a flow diagram of additional details of the exemplary algorithm or method for test report calculation of FIG. 6 in accordance with various embodiments;
FIG. 8 is a block diagram of an exemplary cloud-based system configuration in accordance with various embodiments;
FIG. 9 is a block diagram illustrating communication with a local or cloud-based system in accordance with various embodiments;
FIG. 10 is a block diagram of an exemplary local manager in accordance with various embodiments; and
FIG. 11 is a block diagram of an exemplary cloud-based system in accordance with various embodiments.

### DETAILED DESCRIPTION

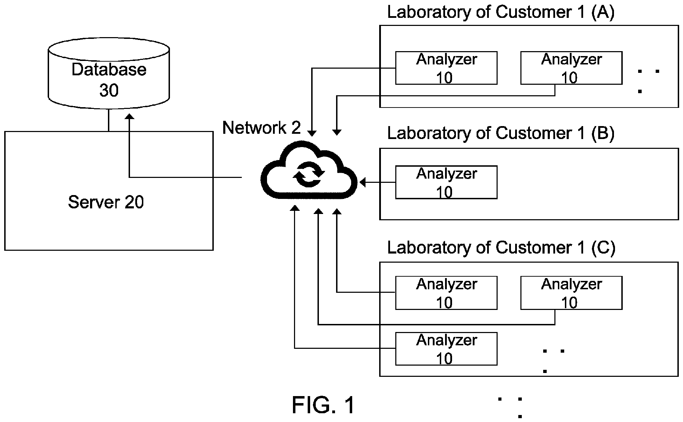

FIG. 1 is a block diagram of an exemplary system configuration in accordance with various embodiments. A system may include a server 20, a database 30, and analyzers 10. The server 20 (optionally along with the database 30) may comprise a computing system. As discussed below, the server 20 may further comprise a controller. The analyzers 10 may be deployed in a customer's laboratory 1. Each laboratory 1 may include a single or a plurality of analyzers 10. The analyzers 10 may be connected to a network 2. The network 2 may be the Internet. The network 2 may be established as a virtual private network. The analyzers 10 may be connected to the server 20 (e.g., connected to the computing system and/or the controller) via the network 2. The analyzers 10 send a result of a measurement to the server 20 via the network 2. The server 20 may store the received results in the database 30. Also, the server 20 may execute a plurality of determination rules defined by the database 30 to determine a count of reportable sample test results that are to be charged. The determination rules may be used for selecting the reportable sample test result. The rules may be adaptable to each of a plurality of customers. The rules may be adaptably defined for each customer so that, for example, the rules may be defined differently for each customer to suit individual situations (number and types of frequent sample tests) in the laboratory of each customer. The database 30 may also store the plurality of determination rules for each customer. The determination rules may be prepared or produced based on a contract between each customer and the provider of the sample analyzers 10. Contracts may be different among customers. Therefore, the plurality of determination rules may also be different among customers. The determination rules may be prepared or produced based on arrangements, other than the contract, between each customer and the analyzer provider. In various examples, multiple laboratories (e.g., 1A, 1B, and 1C) may exist, each of which may be associated with the same or different customers, and which may also include one or more analyzers 10.

The customer may have a contract with a provider of the analyzer 10. The contract may define that the customer shall pay a certain price for a number of reportable test results measured by the analyzer 10. The payment may mean a monthly payment. The server 20 calculates, based on the results sent from the analyzers 10, the number of reportable test results for determining the billing amount for the customer.

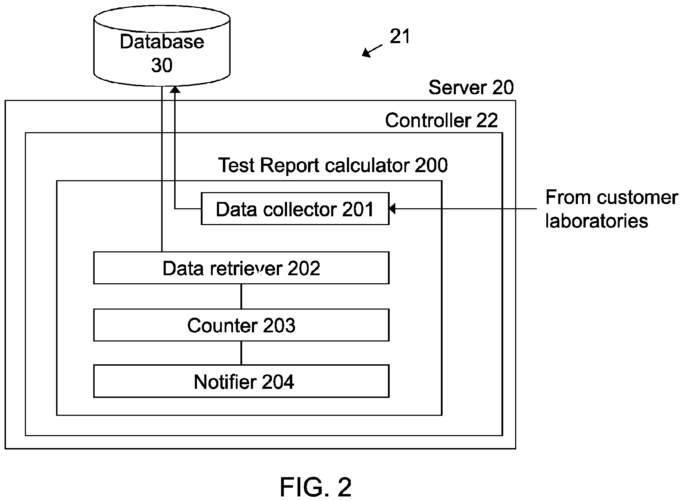

FIG. 2 is a block diagram of an exemplary computing system 21 in accordance with various embodiments. The computing system 21 may include the server 20 and, optionally, the database 30. The server 20 may include controller 22, which may in turn include a test report calculator 200. The test report calculator 200 calculates a number of reportable test results. Based on the number of the reportable test results, a billing amount to a customer for a certain period (e.g., monthly) can be determined. The reportable test result may be a measurement result that can be reported to a doctor or physician who issued an order for a test, or reportable to a billing entity (e.g., an insurance company). For example, the test result may include an erroneous result or a result for a quality control sample measurement. Such result may not be reported to the doctor or physician or the billing entity. If such non-reportable test results are included in a calculation process for the billing amount, the calculated billing amount may not be accurate. Instead, the test report calculator 200 according to the present disclosure accurately calculates the reportable test result by excluding the non-reportable test result in the calculation process. The aforementioned determination rules may be adaptably defined to determine, for each of the customers, whether to select or exclude such test result. It may depend on each customer which test result is to be selected to be charged and which test result is to be excluded so as not to be charged. For example, the test results such as the erroneous result or the result for the quality control sample measurement may be selected to be charged for a customer while such test results may be excluded so as not to be charged for other customers.

The test report calculator 200 may include a data collector 201, a data retriever 202, a counter 203, and a notifier 204. The data collector 201 receives the test results from the analyzers 10 and stores the received results in the database 30. For example, the data collector 201 may receive a first plurality of test results from one or more sample analyzers 10 in a first laboratory (e.g., laboratory 1A) of a first customer, and may receive a second plurality of test results from one or more sample analyzers 10 in a second laboratory (e.g., laboratory 1B) of a second customer. The data collector 201 may receive the plurality of test results from the one or more sample analyzers 10 either periodically (e.g., once a day, once an hour, etc.) or every time the one or more sample analyzers 10 perform a sample testing. The data retriever 202 retrieves, from the database 30, necessary information for the calculation process for the number of reportable test results or the billing amount. The counter 203 calculates the number of reportable test results based on the retrieved information. The notifier 204 notifies the customer of the calculated billing amount. Alternatively, the notifier 204 notifies another system (such as an enterprise system) of a calculated number of reportable test results, wherein the other system will calculate a billing amount for the customer based on that calculated number of reportable test results.

In accordance with various embodiments, a computing system 21 may include the server 20 and the database 30. The server 20 may comprise a controller 22, which may be configured to perform various functions and/or method steps as described below and herein. The server 20, and/or the controller 22, may include one or more processing devices coupled to one or more memories, for example, with a data bus. The processing device may be a Central Processing Unit (CPU), microcontroller, or a microprocessor, and/or may include or be implemented with an Application Specific Integrated Circuit (ASIC), Programmable Logic Device (PLD), or Field Programmable Gate Array (FPGA); or as circuitry that includes discrete logic or other circuit components, including analog circuit components, digital circuit components or both; or any combination thereof. The circuitry may include discrete interconnected hardware components or may be combined on a single integrated circuit die, distributed among multiple integrated circuit dies, or implemented in a Multiple Chip Module (MCM) of multiple integrated circuit dies in a common package, as examples. The memory may comprise a flash memory, a Random Access Memory (RAM), a Read Only Memory (ROM), an Erasable Programmable Read Only Memory (EPROM), a Hard Disk Drive (HDD), other magnetic or optical disk, or another machine-readable nonvolatile medium or other tangible storage mediums other than a transitory signal. The memory may store therein software modules and instructions that, when executed by the processing device or the controller 22, cause the processing device or the controller 22 to implement any of the processes described herein or illustrated in the drawings. The memory may also store other data for use by the processing device or controller 22. The server 20, and thus, the controller 22, may include one or more network interfaces configured to communicate over a network. Additionally, the analyzers 10 may include a similar structure, including a processor, memory, storage, and a network interface. In this manner, the server 20 or controller 22 may communicate with the analyzers 10 via a network.

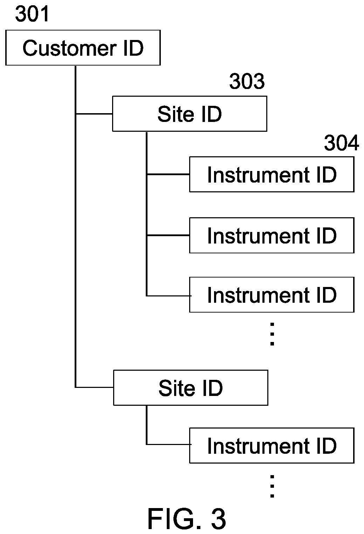

FIG. 3 is a block diagram of an exemplary data structure of a database 30 in accordance with various embodiments. The database 30 may be configured by a relational database (RDB). The database 30 stores data representing list of analyzers 10 deployed in the customers' laboratory 1 or laboratories 1. The customer ID 301 may represent a customer. The site ID 303 and instrument ID 304 may represent the customer's laboratory 1 and analyzers 10 deployed in each laboratory 1, respectively. The instrument ID 304 may represent various analyzers 10 such as hematology analyzers, smear slide makers, urine analyzers, immunology analyzers, blood coagulation analyzers, or gene testing analyzers. That is, in various embodiments, the data shown in FIG. 3 may represent a laboratory configuration of the customer per customer ID.

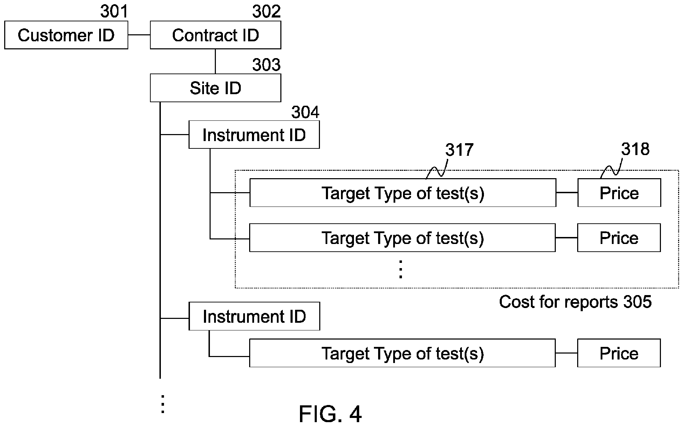

FIG. 4 is a block diagram of an exemplary data structure which may represent the contract between the customer and the provider of the analyzer in accordance with various embodiments. The contract ID 302 may represent the contract between the customer and the provider of the analyzer 10. For example, the analyzer 10 corresponding to at least one of the instrument ID 304 is a hematology analyzer. For example, the hematology analyzer may be able to measure following measurement items: RBC, HCT, MCV, MCH, MCHC, PLT, MPV, WBC, NRBC#, NRBC%, BASO#, BASO%, HGB, WBC, NEUT#, NEUT%, LYMPH#, LYMPH%, MONO#, MONO%, EO#, EO%, IG#, IG%, RET#, RET%, PLT, IRF, RET-He, etc. In various embodiments, as shown in FIG. 4, the contract ID 302 may be associated with each instrument ID 304 via a particular site ID 303 at which the analyzer corresponding to the instrument ID 304 is installed. In other approaches, the contract ID 302 may be directly associated with each instrument ID 304.

In various examples, the contract may define the payment structure and arrangement for the measurements by the analyzer 10 according to various measurement items. The contract may include cost information 305 for various reports, which may include one or more prices 318 and target type of tests 317. The price 318 may represent an amount of payment per measurement. The price 318 may be associated with a target type of test(s) 317. The target type of test(s) 317 may contain a list of measurement items subjected to the price 318. In some examples, some target type of test(s) 317 may combine all of or more than one of the measurement items into one price 318. In other examples, some target type of test(s) 317 may correspond to individual measurement item (e.g., the price 318 may be defined for each of the measurement items) or groups of measurement items. In certain approaches, the target type of test(s) 317 may contain a list of some measurement items among all of items. In various examples, the contract may define non-target type of test for which the provider does not bill to the customer. The non-target type of test may include a test for a non-patient sample (such as a quality control sample, a calibrator sample, a fluid used for a background check), a test that are associated with an error (such as a measurement error, a sample ID read error), duplicate test (such as a rerun test, reflex test). The aforementioned determination rules may be adaptably defined to determine, for each of the customers, which type of test is subjected to a target of the billing. It may depend on each customer which type of test is to be defined as the target type of test and which type of test is to be defined as the non-target type of test. Therefore, tests such as the test for the non-patient sample, the test associated with the error and the duplicate test may be defined as the target type of test for a customer while such tests may be defined as the non-target type of test for other customers. It may also depend on each customer which sample is defined as a sample for the non-target type of test among the non-patient samples (such as the quality control sample, the calibrator sample, the fluid used for the background check), or which error is defined as an error for the non-target type of test among the errors (such as the measurement error, the sample ID read error), or which test is defined as a duplicate test for the non-target type of test among the tests (such as the rerun test, reflex test). The target type of test and the non-target type of test may be adaptably defined for each customer.

The contract may be established per discipline such as hematology, urine, blood coagulation, immunology, etc. For example, between the customer and the provider of the analyzer 10, the contract may be established for the hematology discipline. In such an exemplary case, the instrument ID 304 associated with the contract ID 302 may represent an analyzer 10 for hematology testing, and the target type of test 317 may represent measurement items for hematology testing.

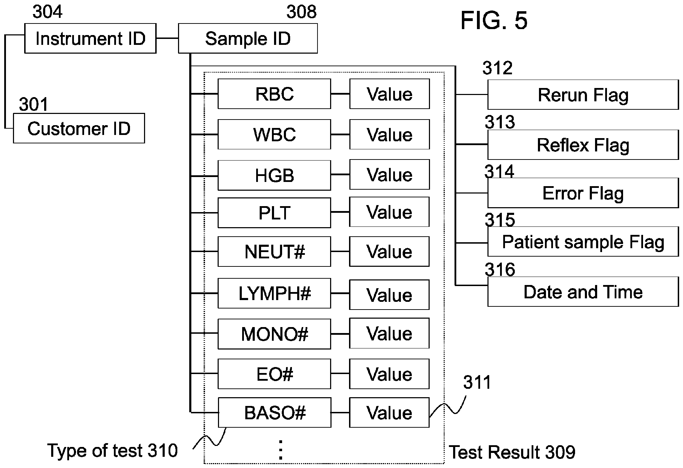

FIG. 5 is a block diagram of an exemplary data structure of a measurement result in accordance with various embodiments. The test result 309 may represent the measurement result of the analyzer 10 which is represented by the instrument ID 304. The test result 309 is associated with the sample ID 308 and the instrument ID 304. The sample ID 308 and instrument ID 304 are associated with the customer ID 302. The test result 309 may comprises a type of test 310 and, optionally, a value 311 associated with the type of test 310. The type of test 310 may represent one of the measurement items. In the exemplary data structure shown in the FIG. 5, the test result 309 represents a result of a hematology test, though other types of tests are possible. The value 311 may represent a measured valued of the measurement item (e.g., test item) subjected to a test order.

The test result 309 may be associated with one or more flags. Each flag may include specific information associated with the flag, such as which test generated the flag or other information. In various embodiments, the rest result 309 may be associated with a rerun flag 312, a reflex flag 313, an error flag 314, a patient sample flag 315, and/or a date and time 316. The rerun flag 312 identifies whether the test result 309 is a result of a rerun test. The analyzer 10 may set the rerun flag 312 flag as "1" if the rerun test is issued, and may associate it with a test result corresponding to a measurement which causes the rerun test, and then may send the test result with this flag to the server 20. The reflex flag 313 identifies whether the test result 309 is a result of a reflex test. The analyzer 10 may set the reflex flag 313 as "1" if the reflex test is issued, and associate it with a test result corresponding to a measurement which causes the reflex test, and then may send the test result with this flag to the server 20. The error flag 314 identifies whether the test result 309 is an erroneous result. The analyzer 10 may set the error flag 314 as "1" if the error (e.g., a measurement error) occurs, and may associate it with corresponding test result, and then may send the test result with this flag to the server 20. The measurement error may relate to mixing, blood too thick, or other conditions with the patient's blood where the analyzer's internal verification process did not allow a result. The patient sample flag 315 identifies whether the test result 309 is a result for a measurement of a non-patient sample, such as quality control sample, a calibrator sample, a fluid used for background check, etc. The analyzer 10 may set the patient sample flag 315 as "0" if, for example, a measurement is performed for a predetermined sample such as a QC sample, a calibrator sample, or the fluid used for background check, etc. The analyzer 10 may set the patient sample flag 315 as "1" if the measurement is performed for a patient sample. The analyzer 10 may send the test result with the patient sample flag to the server 20. The date and time 316 represents a date and time of the measurement. Other flags may be possible, as well. For example, a LIS (Laboratory Information System) flag may indicate whether or not the test result 309 was sent to the LIS for the laboratory 1.

Referring briefly to FIG. 2, in various approaches, the data collector 201 of the server 20 may receive, from the analyzer 10, information corresponding to the sample ID 301, the instrument ID 304, the test result 309, flags 312-315, and the date and time 316. The data collector 201 may associate the received information with the customer ID 301 and then store the information into the database 30. The data collector 201 may recognize the corresponding customer ID 301 to be associated with the received information based on a sender's address (e.g., IP address of the sender). The database 30 may contain relation information between the customer ID 301 and the sender's address, and the data collector 201 may refer to the database 30 and recognize corresponding customer ID 301 based on the sender's address.

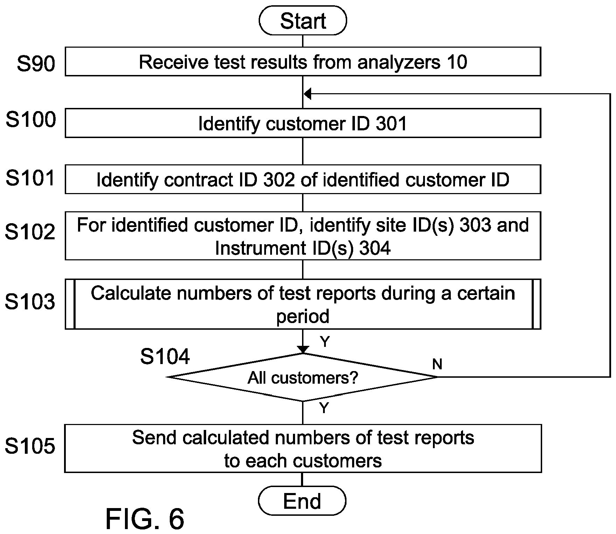

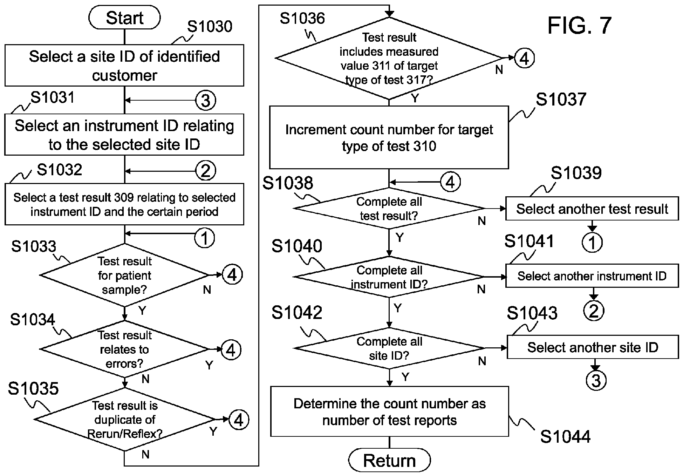

FIG. 6 is a flow diagram of an exemplary algorithm or method for test report calculation in accordance with various embodiments. In various embodiments, the server 20, the controller 22, the computing system 21, generally, and/or the analyzers 10 are configured and/or programmed to perform the various steps depicted in the method of FIG. 6 (and in FIG. 7, described below). In accordance with various embodiments, the test report calculator 200 of the server 20 calculates the number of reportable test results according to the exemplary algorithm shown in FIGS. 6 and 7.

At S90, the data collector 201 receives the test results from the analyzers 10 and stores the received results in the database 30. For example, the data collector 201 may receive a first plurality of test results from one or more sample analyzers 10 in a first laboratory (e.g., laboratory 1A) of a first customer, and may receive a second plurality of test results from one or more sample analyzers 10 in a second laboratory (e.g., laboratory 1B) of a second customer. The data collector 201 may receive the plurality of test results from the one or more sample analyzers 10 either periodically (e.g., once a day, once an hour, etc.) or every time the one or more sample analyzers 10 perform a sample testing.

At S100, the data retriever 202 of the server 20 retrieves data relating to the test report calculation. For example, the data retriever 202 retrieves data relating to or indicating (e.g., listing) customers subjected to the test report calculation. Then, the counter 203 identifies a customer ID 301 corresponding to a customer subjected to the test report calculation. After the test report calculation for the identified customer ID 301 is completed, then the counter 203 identifies another customer ID 301 corresponding to another customer subjected to the test report calculation. This process may repeat until test report calculations for all customer IDs subject to the test report calculation are completed.

At S101. The counter 203 identifies a contract ID 302 associated with the identified customer ID 301. If a plurality of contract IDs 312 are associated with the identified customer ID 301, the counter 203 then identifies those contract IDs 302.

At S102, the counter 203 identifies a site ID 303 associated with the customer ID 301, and then identifies an instrument ID 304 associated with the identified site ID 303. If a plurality of site IDs 303 are associated with the identified customer ID 301, the counter 203 identifies these site IDs 303. If a plurality of instrument IDs 304 are associated with the identified site ID 303, the counter 203 identifies these instrument IDs 304. For example, the counter 203 identifies all site IDs 303 and instrument ID 304 corresponding to the customer ID 301. As discussed above, the contract may associate the sample analyzer (e.g., instrument ID 304), one or more test items (e.g., target type of tests 317) subjected to a target of the billing, and a price 318 per testing.

At S103, the counter 203 performs a process for calculating the test report calculation. An exemplary algorithm or method for step S103 is shown explained below with respect to FIG. 7.

At S104, after the counter 203 completes the test report calculation for a certain customer, then the counter 203 may move to the calculation process for another customer. The counter 203 repeats the calculation process until the process for all customers subjected to the test report calculation is completed. Alternatively, the counter 203 may perform the calculation process for one or some customers, one or some locations, or one or some analyzer.

At S105, the notifier 204 sends a calculated number of the reportable test results to customers or to another computing system such as an enterprise system. The notifier 204 may send a notification including the calculated number to the customers. The notifier 204, for example, identifies correspondences (e.g., e-mail address) of the customers based on the information stored in the database 30 and then may send the notification to the identified correspondences.

FIG. 7 is a flow diagram of additional details of the exemplary algorithm or method for test report calculation of FIG. 6 in accordance with various embodiments. In particular, FIG. 7 illustrates additional details of the step S103 of FIG. 6 for calculating the number of test reports during a certain period. Specifically, FIG. 7 illustrates details of a method for selecting test results from the plurality of test results, based on a plurality of determination rules (e.g., S1033 - S1036) adapted to the customer identified at S100 which are applied to the test result, and a method for determining a number of the selected test results for a billing to the customer. As stated above, the server 20, the controller 22, the computing system 21, generally, the counter 203, and/or the analyzers 10 are configured and/or programmed to perform the various steps depicted in the method of FIG. 7.

At S1030, the counter 203 selects the site ID 317 identified in step S102. If a plurality of site IDs are identified in the step S102, the counter 203 selects one of the site IDs 317 identified in S1030. At S1031, the counter 203 selects the instrument ID 304 associated with the selected site ID 317. If a plurality of instrument IDs are associated with the selected site ID 317, the counter 203 selects one of the instrument IDs 304 in step S1031. In this step, the counter 203 selects an instrument ID 304 which has not yet been selected.

At S1032, the counter 203 selects the test result 309 associated with the selected instrument ID 304. The counter 203 selects the test result 309 which corresponds to a measurement by the analyzer 10 during the certain period (e.g., a test result 309 measured within a month subject to the test report calculation). If a plurality of test results 305 are associated with the selected instrument ID 304, the counter 203 selects one of the test results 305 in this S1032 step. In this step, the counter 203 selects a test result 309 that has not yet been selected. In this manner, the method further includes selecting test results based on the contract, the plurality of determination rules, and the analyzer identification information for identifying the one or more analyzers which are installed in the laboratory.

In the following steps (i.e., steps S1033-S1036), the counter 203 tests or compares each test result according to a set of determination rules to determine whether to count them as a reportable test result or to exclude them from the count of reportable test results. If, after being tested or compared to the determination rules, the counter 203 determines that the test result is not to be excluded, the counter 203 increments a count number that corresponds to the number of reportable test results. In one example, the determination rules can be considered as being for selecting test results which are reportable to a medical doctor or a billing entity (e.g., an insurance company or other entity that ordered the test). In another example, the determination rules can be considered as being for excluding a test result that is not reportable to a medical doctor or a billing entity. In various embodiments, the determination rules can comprise first, second, and/or third rules (or more), which are discussed in further detail below.

At S1033, the counter 203 examines whether the test result 309 is a result of the measurement for a non-patient sample (such as quality control sample, a calibrator sample, a fluid used for background check) or not. Stated another way, the counter 203 applies a first determination rule for selecting the test results of a sample collected from a subject. The first determination rule thus may be for excluding the test result of a quality control sample, a calibrator sample or a fluid for background check. For example, the counter 203 examines, based on the patient sample flag 315, whether the test result 309 is a result of the measurement for a non-patient sample or not. If it is not a patient sample, the counter 203 skips to step S1038, and thus does not increment the count number for the target type of test. However, if counter 203 does determine that the test sample is a patient sample, the counter 203 then moves to step S1034. Thus, by performing step S1033, the counter 203 excludes test results 309 associated with the non-patient samples (such as a QC sample or a calibration sample) from the counting process of the reportable test results. It may be adaptable to each customer whether to apply the first determination rule to the test results. The first determination rule may not be applied when determining a count of reportable test results for some customers.

At S1034, the counter 203 examines whether the test result 309 relates to an error or not. Stated another way, the counter 203 applies a second determination rule for excluding test results that are associated with an error. For example, the counter 203 examines, based on the error flag 314, whether the test result 309 relates to an error or not. If it is a test result relating to an error, the counter 203 skips to step S1038, and thus does not increment the count number for the target type of test. However, if the test result is not related to the error, the counter 203 then moves to step S1035. Thus, by performing step S1034, the counter 203 excludes test results 309 relating to the error from the counting process of the reportable test results (e.g., the erroneous result may not be suitable for reporting to the doctor/physician or billing entity). It may be adaptable to each customer whether to apply the second determination rule to the test results. The second determination rule may not be applied when determining a count of reportable test results for some customers.

At S1035, the counter 203 examines whether the test result 309 is a duplicate of the rerun or reflex test. Stated another way, the counter 203 applies a third determination rule for excluding test results that are a duplicate of a retesting, and/or for excluding test results corresponding to a test item that is the same as that of the retesting. For example, the counter 203 examines, based on the rerun flag 308 and/or the reflex flag 313, whether the test result 309 is the duplicate of the rerun or reflex test. If the test result 309 is a duplicate of the rerun or reflex test, the counter 203 skips to step S1038, and thus does not increment the count number for the target type of test. However, if the test result 309 is not a duplicate, the counter 203 moves to step S1036. Thus, by performing step S1035, the counter 203 excludes the test result 309 which are duplicate of a rerun or reflex test from the counting process of the reportable test results (e.g., such duplicative test result may not be suitable for reporting to the doctor/physician or billing entity). The counter 203 may examine whether the test result 309 is a duplicate of the rerun or reflex test by checking the sample ID. If the same Sample ID already exists in the Database 30, the counter 203 may determine the test result 309 is duplicate. It may adaptable to each customer whether to apply the third determination rule to the test results. The third determination rule may not be applied when determining a count of reportable test results for some customers.

At S1036, the counter 203 may examine whether the test result 309 includes the value 311 of the target type of test 317 associated with the customer ID identified at S100. For example, the counter 203 examines whether at least one of the type of tests 317 of the test result 309 corresponds to the measurement items listed in the target type of test 317. The counter 203 may identify the target type of test 317 according to the instrument ID 304 selected at step S1031 (as shown in Fig. 4, the target type of test 317 is associated with the instrument ID 304). If the test result 309 includes the value 311 of the target type of test 317, the counter 203 performs step S1037 by incrementing the count. If the test result 309 does not include the measured value 311 of the target type of test 317, the counter 203 skips to step S1038, and thus does not increment the count number for the target type of test. For example, by performing step S1036, the counter 203 excludes test results 309 that do not include the result subjected to the contract. For example, the test result 309 relating to the selected instrument ID 304 may be related to a discipline not corresponding to the contract ID 302, therefore, such test result 309 may be excluded from the counting process of the reportable test results since the test result 309 does not correspond to the contract. The measurement items listed in the target type of test 317 may be different among customers. Therefore, at S1036, the counter 203 executes or implements a determination rule adapted to the customer identified at S100 to examine whether a test result corresponds to the measurement items listed in the target type of test 317.

At S1037, the counter 203 determines that the test result 309 is to be included in a count of reportable test results. Put another way, the controller 203 determines, according to the determination rules for excluding a test result, that a first test result of the plurality of test results is not to be excluded. Accordingly, the counter 203 increments a count number for the target type of test 317. In one example, a count number is provided for each of the target type of test 317. In other examples, a count number is provided for sets or groups of target types of tests 317. In another example, one count number is provided for all target types of tests 317.

At S1038, the counter 203 examines whether all test results 309 have been examined. For example, a plurality of test results 309 are associated with the instrument ID 304 selected at S1031. The counter 203 examines if all of the plurality of test results 309 have been examined though one or all of steps S1033-S1036. If not, the counter 203 moves to step S1039. If the counter 203 has examined all of plurality of test results 309 for an instrument ID, the counter 203 moves to step S1040.

At S1039, the counter 203 selects another test result 309. The counter 203 selects a test result 309 that it has not selected yet. Then, the counter 203 moves back to step S1033 to repeat the process with the newly selected test result 309.

At S1040, the counter 203 examines whether all instrument IDs 304 have been examined. For example, a plurality of instrument IDs 304 may be associated with the site ID 303 selected at step S1030. The counter 203 examines if all of the plurality of instrument IDs 304 (really, all of the test results 309 of all of the plurality of instrument IDs 304) have been examined through one or more of the steps S1031-S1040. If not, the counter 203 moves to step S1041 to select another instrument ID. If the counter 203 has examined all of the plurality of instrument IDs 304, then the counter 203 moves on to step S1042.

At S1041, the counter 203 selects another instrument ID 304. The counter 203 selects an instrument ID 304 that has not yet been selected. Then, the counter 203 returns back to step S1032 to repeat the process with the newly selected instrument ID 304.

At S1042, the counter 203 examines whether all site IDs 303 have been examined. For example, a plurality of site IDs 303 may be associated with the customer ID 301 identified at step S100 (in FIG. 6). The counter 203 examines if all of the plurality of site IDs 303 have been examined though one or more of steps S1030-S1042. If not, the counter 203 moves to step S1043. If the counter 203 has examined all of the plurality of site IDs 303, then the counter 203 moves to step S1044.

At S1043, the counter 203 selects another site ID 303. The counter 203 selects a site ID 303 that has not yet been selected. Then, the counter 203 moves back to step S1031 to repeat the process with the newly selected site ID 303.

At S1044, the counter 203 determines the count number as number of test reports. For example, the count number is provided for each of the target type of test 317 or groups of target types of tests 317. Therefore, the counter 203 may determine the number of the test reports for each of the target type of test 317. In this exemplary case, an amount of the payment is determined per the target type of test 317 (e.g., the price 318 is multiplied by the number of the test reports for the corresponding target type of test 317). In one approach, the counter 203 may determine the amount of the payment per instrument ID 304. In this exemplary case, the counter 203 summarizes the number of test reports corresponding to the instrument ID 304. The counter 203 may determine the amount of the payment per site ID 303. In this exemplary case, the counter 203 summarizes the number of test reports corresponding to the site ID 303. The counter 203 may determine the amount of the payment per customer ID 301. In this exemplary case, the counter 203 summarizes the number of test reports corresponding to the site IDs 303 and the instrument IDs 304 for the customer ID 301. In another embodiment, the counter 203 may simply communicate, e.g., via the notifier 204, another computer system (such as an enterprise system) of the calculated count of the test reports corresponding to each instrument ID 304, each site ID 303, and/or each customer ID 301. Thereafter, the other computer system may be tasked with determining a final bill based on the count, the contract, and/or one or more other factors.

Returning to FIG. 6, at S104, the counter 203 may determine if there are any other customer IDs 301 that require counting. If so, the counter 203 will repeat the above process for the test results associated with that customer ID 301. Specifically, the counter 203 may select second test results from the second plurality of test results received from the one or more sample analyzers in the second laboratory, based on a second plurality of determination rules adapted to the second customer, which are applied to the second test results. The second plurality of determination rules may be different from the first plurality of determination rules. The counter 203 may then determine a number of the selected second test results for a billing to the second customer. This process can repeat again for a third and other customer IDs 301. Accordingly, an accurate count of reportable test results for one or all customers can be determined.

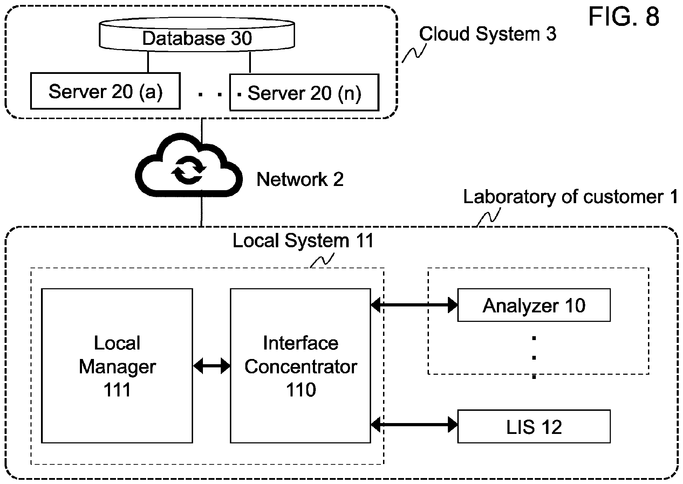

In various embodiments, the above described methods and systems may be implemented in a cloud-based system. FIG. 8 is a block diagram of an exemplary cloud-based system configuration in accordance with various embodiments. A cloud system 3 is connected over the network 2 to the laboratory 1 of the customer. The laboratory 1 may include a local system 11, the analyzer(s) 10, and a laboratory information system (LIS) 12. The local system 11 may include a local manager 111 that acts as an agent for transferring data back and forth between an interface concentrator 110 and a server 20 of the cloud system 3. The local manager 111 may be the same device having functions of the interface concentrator 110, or may be separate from the interface concentrator 110. The interface concentrator 110 may simplify interfaces between the analyzer(s) 10 and minimize the LIS 12 connectivity to a single interface. The single interface may use, for example, ASTM, HL7 or the like.

The cloud system 3 may have a plurality of servers 20 (e.g., servers 20a-20n). The servers 20 may be implemented in a data center. The details of the server 20 are described in the Fig. 2 and corresponding descriptions. The cloud system 3 may be accessible from a web browser deployed in a user terminal such as a computer, PC, tablet, smartphone, etc. The cloud system 3 may be accessible by multiple laboratories 1 and accessible from anywhere having Internet access. The cloud system 3 can consolidate multiple test results 309 from multiple laboratories 1.

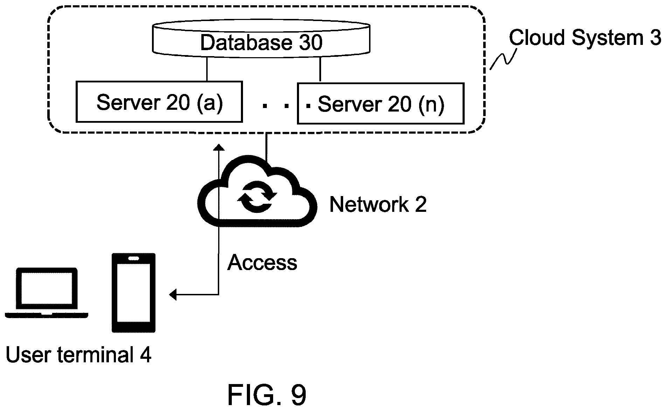

FIG. 9 is a block diagram illustrating communication with a local or cloud-based system in accordance with various embodiments. The system shown in FIG. 9 illustrates an exemplary embodiment for communication of a user terminal 4 with the cloud system 3. The user of the user terminal 4 may be, in various embodiments, laboratory workers accessing the test results 309 and the amount of payment. The communication by the user terminal 4 with the cloud system 3 may be through the network 2. The user of the user terminal 4 may access to the cloud system 3 via the web browser or other software of the user terminal 4. The user terminal 4 may be a computer, PC, tablet or smartphone and may include an input device, a display, or a camera which constitutes a user interface. The input device may be a keyboard, touch panel, keypad, a cursor control device, etc.

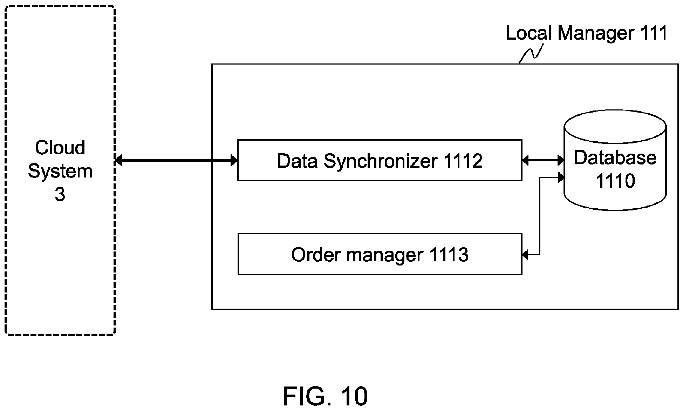

FIG. 10 is a block diagram of an exemplary local manager in accordance with various embodiments. The local manager 111 may include a data synchronizer 1112 that transfers data to the servers 20 of the cloud system 3. The data synchronizer 1112 also receives data from the cloud system 3. The data synchronizer 1112 may also transfer the test result 309 sent from the analyzer 10 to the cloud system 3. The data synchronizer 1112 may transfer/receive the data to/from the cloud system 3 automatically and without user intervention. An IP address may be assigned to the local manager 1112. The cloud system 3 may recognize the IP address assigned to the local manager 1112. The cloud system 3 then allows access via the IP address. The data synchronizer 1112 uses the IP address to communicate with the cloud system 3. The data synchronizer 1112 may operate in a background to be automated and without user intervention. The data synchronizer 1112 may be able to transfer the test result 309 to the cloud system 3 without intervention since it operates in a background of the laboratory system. The operation in the background may mean that the data synchronizer 1112 automatically receives the test result 309 from the analyzer 10 without the user intervention. The operation in the background may mean that the data synchronizer 1112 automatically transfers the test result 309 to the cloud system 3 without the user intervention.

The local manager 1112 may include an order manager 1113. The order manager 1113 manages a test order, which may be issued by the LIS 12 or the cloud system 3. The LIS 12 or the cloud system 3 may issue a rerun/reflex order. The order manager 1113 sends, to the analyzer 10, the test order and the rerun/reflex order.

The database 1110 may store information relating to the test order and the test result 309. The database 1110 may store information relating to the test result 309 sent from the analyzer 10. The database 1110 may store information relating to the test order sent from the LIS 12 or the cloud system 3. As information relating to the test result 309, the database 1110 may store: the sample ID 308, the instrument ID 304, the type of test 310, the value 311, the rerun flag 312, the reflex flag 313, the error flag 314, the patient sample flag 315, the date and time 316. The data synchronizer 1112 may transfer the above mentioned information to the cloud system 3.

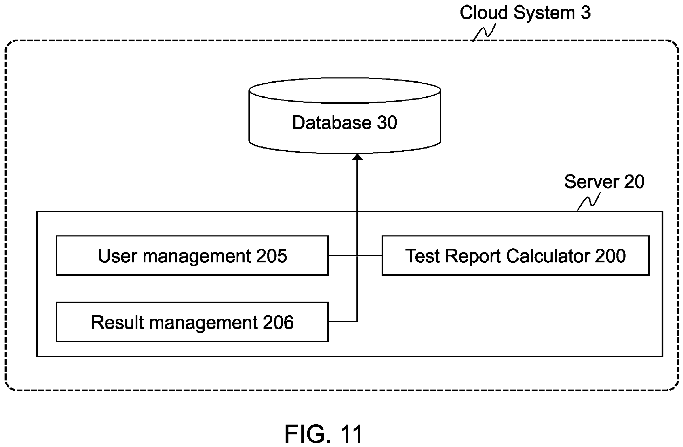

FIG. 11 is a block diagram of an exemplary cloud-based system in accordance with various embodiments. The server 20 may include the test report calculator 200 as shown in FIG. 2, a user management 205, and a result management 206.

The user management 205 manages user accounts which are allowed to access to the cloud system 3. The user management 205 manages a user's login according to account information (e.g., a user name, a password, information for biometric authentication, etc) stored in the database 30. When a user accesses the cloud system 3 for logging in through the web browser of the user terminal 4, the user management 205 may require a user ID and a password to allow the user to log in.

The result management 206 may provide a GUI which allows a user to manage the test results sent from the analyzer 10 via the local manager 111. The result management 206 may provide the GUI to the user terminal 4. The user may be able to view the test result and the amount of the payment relating to the test result via the GUI shown in the user terminal 4. In this exemplary case, the notifier 204 of the test report calculator 200 may provide the amount of the payment calculated by the counter 203 to the GUI shown in the user terminal 4.

So configured, and in accordance with the various methods, systems, and embodiments disclosed above, various technical advantages are realized. Primarily, accurate reportable test result counts are possible, which account for test results that cannot otherwise be counted. As such, accurate and transparent client billing can be achieved.

The description and accompanying drawings above provide specific example embodiments and implementations. The described subject matter may, however, be embodied in a variety of different forms and, therefore, covered or claimed subject matter is intended to be construed as not being limited to any example embodiments set forth herein. A reasonably broad scope for claimed or covered subject matter is intended. Among other things, for example, subject matter may be embodied as methods, devices, components, systems, or non-transitory computer-readable media for storing computer codes. Accordingly, embodiments may, for example, take the form of hardware, software, firmware, storage media, or any combination thereof. For example, the method embodiments described above may be implemented by components, devices, or systems including memory and processors by executing computer codes stored in the memory.

Throughout the specification and claims, terms may have nuanced meanings suggested or implied in context beyond an explicitly stated meaning. Likewise, the phrase "in one embodiment" as used herein does not necessarily refer to the same embodiment and the phrase "in another embodiment" as used herein does not necessarily refer to a different embodiment. It is intended, for example, that claimed subject matter includes combinations of example embodiments in whole or in part.

In general, terminology may be understood at least in part from usage in context. For example, terms, such as "and", "or", or "and/or," as used herein may include a variety of meanings that may depend at least in part on the context in which such terms are used. Typically, "or" if used to associate a list, such as A, B or C, is intended to mean A, B, and C, here used in the inclusive sense, as well as A, B or C, here used in the exclusive sense. In addition, the term "one or more" as used herein, depending at least in part upon context, may be used to describe any feature, structure, or characteristic in a singular sense or may be used to describe combinations of features, structures or characteristics in a plural sense. Similarly, terms, such as "a," "an," or "the," may be understood to convey a singular usage or to convey a plural usage, depending at least in part upon context. In addition, the term "based on" may be understood as not necessarily intended to convey an exclusive set of factors and may, instead, allow for existence of additional factors not necessarily expressly described, again, depending at least in part on context.

Reference throughout this specification to features, advantages, or similar language does not imply that all of the features and advantages that may be realized with the present solution should be or are included in any single implementation thereof. Rather, language referring to the features and advantages is understood to mean that a specific feature, advantage, or characteristic described in connection with an embodiment is included in at least one embodiment of the present solution. Thus, discussions of the features and advantages, and similar language, throughout the specification may, but do not necessarily, refer to the same embodiment.

## Claims

1. A method implemented by a computing system, comprising:
receiving, via network, a plurality of test results from at least one sample analyzer of each of a plurality of customers;
selecting test results from the plurality of test results, based on a plurality of determination rules adaptable to each of the customers; and
determining, for each of the customers, a number of the selected test results, wherein
the determination rules are for selecting the test result which is reportable to a medical doctor,
the determination rules comprise a first rule for selecting the test result of a sample collected from a subject,
the first rule is for excluding the test result of a quality control sample, a calibrator sample or a fluid for background check,
the determination rules comprise a second rule for excluding the test result which is associated with an error, and
the determination rules comprise a third rule for excluding the test result which is a duplicate of a retesting.

2. The method of claim 1, wherein
the determination rules are adaptably defined for each of the customers.

3. The method of claim 1 or 2, wherein
the determination rules are adaptably defined to determine, for each of the customers, whether to select or exclude the test result.

4. The method of any one of claims 1 to 3, wherein
the determination rules are for excluding the test result which is not reportable to the medical doctor.

5. The method of claim 4, further comprising:
determining, according to the determination rules for excluding the test result, that a first test result of the plurality of test results is not to be excluded; and
incrementing a count number corresponding to a number of reportable test results.

6. The method of claim 1, wherein
the third rule is for excluding the test result whose test item is the same as that of the retesting.

7. The method of any one of claims 1 to 6, wherein
the test result includes a value for a test item subjected to a test order.

8. The method of any one of claims 1 to 7, wherein
the receiving the plurality of test results comprises receiving the test result from the at least one sample analyzer at least one of periodically or every time the at least one sample analyzer performs a sample testing.

9. A computing system, comprising
a controller connected to at least one sample analyzer of each of a plurality of customers via network, wherein
the controller is programmed to:
receive a plurality of test results from the at least one sample analyzer;
select test results from the plurality of test results, based on a plurality of determination rules adaptable to each of the customers; and
determine, for each of the customers, a number of the selected test results, wherein
the determination rules are for selecting the test result which is reportable to a medical doctor,
the determination rules comprise a first rule for selecting the test result of a sample collected from a subject,
the first rule is for excluding the test result of a quality control sample, a calibrator sample or a fluid for background check,
the determination rules comprise a second rule for excluding the test result which is associated with an error, and
the determination rules comprise a third rule for excluding the test result which is a duplicate of a retesting.

10. The computing system of claim 9, wherein
the determination rules are adaptably defined for each of the customers.

11. A system, comprising:
at least one sample analyzer of each of a plurality of customers; and
a computing system connected to the at least one sample analyzer via network, and programmed to:
receive a plurality of test results from the at least one sample analyzer;
select test results from the plurality of test results, based on a plurality of determination rules adaptable to each of the customers; and
determine, for each of the customers, a number of the selected test results, wherein
the determination rules are for selecting the test result which is reportable to a medical doctor,
the determination rules comprise a first rule for selecting the test result of a sample collected from a subject,
the first rule is for excluding the test result of a quality control sample, a calibrator sample or a fluid for background check,
the determination rules comprise a second rule for excluding the test result which is associated with an error, and
the determination rules comprise a third rule for excluding the test result which is a duplicate of a retesting.

## Patentansprüche

1. Verfahren, das von einem Computersystem implementiert wird, umfassend:
Empfangen, über ein Netzwerk, einer Vielzahl von Testergebnissen von mindestens einem Probenanalysator von jedem einer Vielzahl von Kunden;
Auswählen von Testergebnissen aus der Vielzahl von Testergebnissen auf der Grundlage einer Vielzahl von Bestimmungsregeln, die an jeden der Kunden angepasst werden können; und
Bestimmen, für jeden der Kunden, einer Anzahl aus den ausgewählten Testergebnissen, wobei die Bestimmungsregeln zum Auswählen des Testergebnisses dienen, das einem Arzt zu melden ist,
die Bestimmungsregeln eine erste Regel zum Auswählen des Testergebnisses einer von einem Probanden entnommenen Probe umfassen,
die erste Regel dazu dient, das Testergebnis einer Probe zur Qualitätskontrolle, einer Kalibrierprobe oder einer Flüssigkeit zur Hintergrundkontrolle auszuschließen,
die Bestimmungsregeln eine zweite Regel zum Ausschließen des Testergebnisses umfassen, das einem Fehler zugeordnet ist, und
die Bestimmungsregeln eine dritte Regel zum Ausschließen des Testergebnisses umfassen, das ein Duplikat eines erneuten Tests ist.

2. Verfahren nach Anspruch 1, wobei
die Bestimmungsregeln für jeden der Kunden anpassbar definiert sind.

3. Verfahren nach Anspruch 1 oder 2, wobei
die Bestimmungsregeln anpassbar definiert sind, um für jeden der Kunden zu bestimmen, ob das Testergebnis ausgewählt oder ausgeschlossen werden soll.

4. Verfahren nach einem der Ansprüche 1 bis 3, wobei
wobei die Bestimmungsregeln dazu dienen, das Testergebnis auszuschließen, das dem Arzt nicht gemeldet werden muss.

5. Verfahren nach Anspruch 4, weiter umfassend:
Bestimmen, gemäß den Bestimmungsregeln zum Ausschließen des Testergebnisses, dass ein erstes Testergebnis der Vielzahl von Testergebnissen nicht ausgeschlossen werden soll; und
Inkrementieren einer Zählnummer entsprechend der Anzahl meldepflichtiger Testergebnisse.

6. Verfahren nach Anspruch 1, wobei
die dritte Regel dazu dient, das Testergebnis auszuschließen, dessen Testgegenstand mit dem des erneuten Tests übereinstimmt.

7. Verfahren nach einem der Ansprüche 1 bis 6, wobei
das Testergebnis einen Wert für einen Testgegenstand einschließt, der einem Testauftrag unterzogen wurde.

8. Verfahren nach einem der Ansprüche 1 bis 7, wobei
das Empfangen der Vielzahl von Testergebnissen umfasst, dass das Testergebnis von dem mindestens einen Probenanalysator mindestens entweder in regelmäßigen Abständen oder jedes Mal, wenn der mindestens eine Probenanalysator eine Probenuntersuchung durchführt, empfangen wird.

9. Computersystem, umfassend
eine Steuereinheit, die über ein Netzwerk mit mindestens einem Probenanalysator von jedem einer Vielzahl von Kunden verbunden ist, wobei
die Steuereinheit programmiert ist, um:
eine Vielzahl von Testergebnissen von dem mindestens einen Probenanalysator zu empfangen;
Testergebnisse aus der Vielzahl von Testergebnissen basierend auf einer Vielzahl von Bestimmungsregeln, die an jeden der Kunden angepasst werden können, auszuwählen; und
für jeden der Kunden eine Anzahl der ausgewählten Testergebnisse zu bestimmen, wobei
die Bestimmungsregeln zum Auswählen des Testergebnisses dienen, das einem Arzt zu melden ist,
die Bestimmungsregeln eine erste Regel zum Auswählen des Testergebnisses einer von einem Probanden entnommenen Probe umfassen,
die erste Regel dazu dient, das Testergebnis einer Probe zur Qualitätskontrolle, einer Kalibrierprobe oder einer Flüssigkeit zur Hintergrundkontrolle auszuschließen,
die Bestimmungsregeln eine zweite Regel zum Ausschließen des Testergebnisses umfassen, das einem Fehler zugeordnet ist, und
die Bestimmungsregeln eine dritte Regel zum Ausschließen des Testergebnisses umfassen, das ein Duplikat eines erneuten Tests ist.

10. Computersystem nach Anspruch 9, wobei
die Bestimmungsregeln für jeden der Kunden anpassbar definiert sind.

11. System, umfassend:
mindestens einen Probenanalysator von jedem einer Vielzahl von Kunden; und
ein Computersystem, das über ein Netzwerk mit dem mindestens einen Probenanalysator verbunden ist und programmiert ist, um:
eine Vielzahl von Testergebnissen von dem mindestens einen Probenanalysator zu empfangen;
Testergebnisse aus der Vielzahl von Testergebnissen basierend auf einer Vielzahl von Bestimmungsregeln, die an jeden der Kunden angepasst werden können, auszuwählen; und
für jeden der Kunden eine Anzahl der ausgewählten Testergebnisse zu bestimmen, wobei
die Bestimmungsregeln zum Auswählen des Testergebnisses dienen, das einem Arzt zu melden ist,
die Bestimmungsregeln eine erste Regel zum Auswählen des Testergebnisses einer von einem Probanden entnommenen Probe umfassen,
die erste Regel dazu dient, das Testergebnis einer Probe zur Qualitätskontrolle, einer Kalibrierprobe oder einer Flüssigkeit zur Hintergrundkontrolle auszuschließen,
die Bestimmungsregeln eine zweite Regel zum Ausschließen des Testergebnisses umfassen, das einem Fehler zugeordnet ist, und
die Bestimmungsregeln eine dritte Regel zum Ausschließen des Testergebnisses umfassen, das ein Duplikat eines erneuten Tests ist.

## Revendications

1. Procédé mis en œuvre par un système informatique, comprenant :
la réception, par l'intermédiaire d'un réseau, d'une pluralité de résultats de test provenant d'au moins un analyseur d'échantillon de chaque client parmi une pluralité de clients ;
la sélection de résultats de test parmi la pluralité de résultats de test, sur la base d'une pluralité de règles de détermination adaptables à chacun des clients ; et
la détermination, pour chacun des clients, d'un nombre des résultats de test sélectionnés, dans lequel les règles de détermination sont destinées à sélectionner le résultat de test qui est à communiquer à un médecin,
les règles de détermination comprennent une première règle pour sélectionner le résultat de test d'un échantillon prélevé chez un sujet,
la première règle est destinée à exclure le résultat de test d'un échantillon de contrôle de qualité, d'un échantillon d'étalonnage ou d'un fluide pour un contrôle d'arrière-plan,
les règles de détermination comprennent une deuxième règle pour exclure le résultat de test qui est associé à une erreur, et
les règles de détermination comprennent une troisième règle pour exclure le résultat de test qui est une réplique de la répétition d'un test.

2. Procédé selon la revendication 1, dans lequel
les règles de détermination sont définies de manière adaptative pour chacun des clients.

3. Procédé selon la revendication 1 ou la revendication 2, dans lequel
les règles de détermination sont définies de manière adaptative pour déterminer, pour chacun des clients, s'il faut sélectionner ou exclure le résultat de test.

4. Procédé selon l'une quelconque des revendications 1 à 3, dans lequel
les règles de détermination sont destinées à exclure le résultat de test qui n'est pas à communiquer au médecin.

5. Procédé selon la revendication 4, comprenant en outre :
la détermination, en fonction des règles de détermination pour exclure le résultat de test, qu'un premier résultat de test de la pluralité de résultats de test ne doit pas être exclu ; et
l'incrémentation d'un nombre de comptage correspondant à un nombre de résultats de test à communiquer.

6. Procédé selon la revendication 1, dans lequel
la troisième règle est destinée à exclure le résultat de test dont l'élément testé est le même que celui du nouveau test.

7. Procédé selon l'une quelconque des revendications 1 à 6, dans lequel
le résultat de test inclut une valeur pour un élément testé soumis à une instruction de test.

8. Procédé selon l'une quelconque des revendications 1 à 7, dans lequel
la réception de la pluralité de résultats de test comprend la réception du résultat de test provenant dudit au moins un analyseur d'échantillon, périodiquement et/ou chaque fois que l'au moins un analyseur d'échantillon effectue un test d'échantillon.

9. Système informatique, comprenant
un dispositif de commande connecté, par l'intermédiaire d'un réseau, à au moins un analyseur d'échantillon de chaque client parmi une pluralité de clients, dans lequel
le dispositif de commande est programmé pour :
recevoir une pluralité de résultats de test en provenance dudit au moins un analyseur d'échantillon ;
sélectionner des résultats de test parmi la pluralité de résultats de test, sur la base d'une pluralité de règles de détermination adaptables à chacun des clients ; et
déterminer, pour chacun des clients, un nombre des résultats de test sélectionnés, dans lequel
les règles de détermination sont destinées à sélectionner le résultat de test qui est à communiquer à un médecin,
les règles de détermination comprennent une première règle pour sélectionner le résultat de test d'un échantillon prélevé chez un sujet,
la première règle est destinée à exclure le résultat de test d'un échantillon de contrôle de qualité, d'un échantillon d'étalonnage ou d'un fluide pour un contrôle d'arrière-plan,
les règles de détermination comprennent une deuxième règle pour exclure le résultat de test qui est associé à une erreur, et
les règles de détermination comprennent une troisième règle pour exclure le résultat de test qui est une réplique de la répétition d'un test.

10. Système informatique selon la revendication 9, dans lequel
les règles de détermination sont définies de manière adaptative pour chacun des clients.

11. Système, comprenant :
au moins un analyseur d'échantillon de chaque client parmi une pluralité de clients ; et
un système informatique connecté audit au moins un analyseur d'échantillon par l'intermédiaire d'un réseau, et programmé pour :
recevoir une pluralité de résultats de test en provenance dudit au moins un analyseur d'échantillon ;
sélectionner des résultats de test parmi la pluralité de résultats de test, sur la base d'une pluralité de règles de détermination adaptables à chacun des clients ; et
déterminer, pour chacun des clients, un nombre des résultats de test sélectionnés, dans lequel
les règles de détermination sont destinées à sélectionner le résultat de test qui est à communiquer à un médecin,
les règles de détermination comprennent une première règle pour sélectionner le résultat de test d'un échantillon prélevé chez un sujet,
la première règle est destinée à exclure le résultat de test d'un échantillon de contrôle de qualité, d'un échantillon d'étalonnage ou d'un fluide pour un contrôle d'arrière-plan,
les règles de détermination comprennent une deuxième règle pour exclure le résultat de test qui est associé à une erreur, et
les règles de détermination comprennent une troisième règle pour exclure le résultat de test qui est une réplique de la répétition d'un test.
